Europäisches Patentamt

European Patent Office

Office européen des brevets

(19)

(11) Numéro de publication : **0 611 821 A1**

## (12) DEMANDE DE BREVET EUROPEEN

(21) Numéro de dépôt : **94400302.9**

(22) Date de dépôt : **11.02.94**

(51) Int. Cl.$^5$ : **C12M 1/34**, C12M 1/02

(30) Priorité : **15.02.93 FR 9301675**

(43) Date de publication de la demande :
**24.08.94 Bulletin 94/34**

(84) Etats contractants désignés :
**CH DE ES GB IT LI PT**

(71) Demandeur : **COMMISSARIAT A L'ENERGIE ATOMIQUE**
**31-33, rue de la Fédération**
**F-75015 Paris (FR)**

(72) Inventeur : **Dureault, Bernard**
**126 rue de la Pompe**
**F-75016 Paris (FR)**
Inventeur : **Charles, Yves**
**26 rue Pierre Loti**
**F-92340 Bourg-La-Reine (FR)**
Inventeur : **Quanquin, Michel**
**12 avenue Joseph Kessel**
**F-78180 Montigny-Le-Bretonneux (FR)**
Inventeur : **Vernet, Pierre**
**7 rue des Moulins à vent**
**F-92260 Fontenay-aux-Roses (FR)**

(74) Mandataire : **Des Termes, Monique et al**
**Société Brevatome**
**25, rue de Ponthieu**
**F-75008 Paris (FR)**

(54) **Procédé et dispositif de contrôle de l'activité biologique d'une culture de microorganismes.**

(57)   L'invention concerne un procédé et un dispositif de contrôle de l'activité biologique d'une culture de microorganismes.

Ce procédé consiste à déterminer une condition de culture inconnue (présence de produit toxique, nombre de cellules, concentration en substrat) d'une culture de microorganismes (levures) donnant un dégagement de gaz ($CO_2$), en cultivant cette culture dans le récipient (5) en même temps qu'une culture témoin des mêmes microorganismes dans le récipient (3), dans les mêmes conditions sauf pour l'une d'entre elles, et en déterminant, après une durée appropriée, au moyen du capteur (39), la différence de pression entre les récipients (3) et (5) qui traduit une variation de l'activité biologique entre les deux cultures en raison de la condition de culture différente à déterminer.

EP 0 611 821 A1

La présente invention a pour objet un procédé et un dispositif pour déterminer l'une des conditions de culture d'une culture de microorganismes à partir de l'activité biologique de cette culture.

De façon plus précise, elle concerne la détermination quantitative ou qualitative des conditions de culture suivantes :

- nombre de microorganismes de la culture,
- présence de produits toxiques dans le milieu de culture, et
- concentration du milieu de culture en substrat.

Des microorganismes dont la culture provoque un dégagement de gaz, par exemple des microorganismes capables de réaliser une fermentation éthanolique, tels que les levures de type Saccharomyces, ont déjà été utilisés pour détecter des produits toxiques, minéraux ou organiques dans des effluents, comme il est décrit dans le document WO 92/01064.

Selon ce document, on utilise la propriété inhibitrice des produits toxiques vis-à-vis de la fermentation éthanolique pour détecter la présence de ces produits, en comparant l'activité biologique d'une culture témoin de microorganismes à celle d'une culture de microorganismes effectuée en présence d'un ou plusieurs produits toxiques. Dans ce but, on effectue des cultures du même microorganisme en l'absence et en présence d'un échantillon de l'effluent à contrôler, et on détermine dans chaque cas le volume de gaz carbonique dégagé.

Bien que ce procédé soit satisfaisant, il présente l'inconvénient de ne pas conduire à une bonne fiabilité et une bonne précision car il est difficile de s'affranchir de variations éventuelles de la température et/ou du rythme d'agitation entre la culture témoin et la culture effectuée en présence de l'échantillon.

La présente invention a précisément pour objet un procédé et un dispositif de détermination de l'une des conditions de culture d'une culture de microorganismes, qui permet non seulement d'obtenir une bonne fiabilité et une bonne précision, mais de déterminer de plus d'autres conditions de culture que la présence de produits toxiques.

Selon l'invention, le procédé pour déterminer une condition de culture inconnue d'une culture de microorganismes donnant un dégagement de gaz, comprend les étapes suivantes :

a) cultiver dans un premier récipient de référence fermé de façon étanche, une population desdits microorganismes dans des conditions de culture connues,

b) cultiver dans un second récipient fermé de façon étanche, ayant le même volume que le premier récipient, une population des mêmes microorganismes dans des conditions de culture identiques, sauf pour la condition de culture inconnue, différente,

c) mesurer la différence de pression gazeuse entre le premier et le second récipients, et

d) déterminer à partir de cette différence de pression, la condition de culture inconnue, différente, utilisée dans le second récipient.

Conformément au procédé de l'invention, les deux populations de microorganismes sont placées dans deux récipients distincts et maintenues simultanément dans des conditions de culture rigoureusement identiques sauf pour l'une d'entre elles, telle que la présence de produits toxiques dans le milieu de culture, la concentration en substrat du milieu de culture ou le nombre de microorganismes. Aussi, la mesure, au bout d'un intervalle de temps approprié, de la différence de pression entre les deux récipients traduit la différence d'activité biologique (volumes de gaz dégagés) entre les deux récipients, tout en éliminant les erreurs qui auraient pu être induites par des variations involontaires de certaines conditions de culture telles que la température et le rythme d'agitation.

De préférence selon l'invention, on détermine la différence de pression entre les deux récipients, lorsque la pression dans le premier récipient de référence atteint un seuil prédéterminé qui permet d'observer une différence significative d'activité biologique entre les deux récipients.

La valeur de ce seuil est choisie en fonction des conditions de culture utilisées dans le premier récipient de façon à correspondre à une activité biologique suffisante de la culture de référence et à permettre ainsi la détection d'une différence de pression significative entre les deux récipients.

A titre d'exemple, la valeur de ce seuil peut correspondre à une augmentation de pression dans le premier récipient de $25.10^2$Pa.

Pour mettre en oeuvre le procédé de l'invention, on utilise des microorganismes dont la culture provoque un dégagement de gaz, par exemple des levures capables de réaliser une fermentation éthanolique.

Avec de tels microorganismes, on peut déterminer par le procédé de l'invention les conditions de culture suivantes :

- présence et concentration d'un produit toxique dans le milieu de culture,
- nombre de cellules de microorganismes dans le milieu de culture, et
- concentration en substrat du milieu de culture.

En effet, l'activité fermentaire des microorganismes et en conséquence leur production de gaz, par exemple de $CO_2$, varie pratiquement de façon linéaire avec la concentration en substrat ou en produit toxique du milieu de culture ainsi qu'avec le nombre de cellules du milieu de culture.

De ce fait, une différence de pression entre les deux récipients à condition bien entendu qu'ils aient des volumes identiques, traduit une variation entre la condition de culture inconnue et la condition de culture connue utilisée dans le premier récipient, et peut être utilisée pour déterminer la condition de culture inconnue conformément à l'équation suivante :

$$\frac{C_x}{C_r} = \frac{\Delta P}{P_r}$$

dans laquelle $C_x$ est la condition inconnue, $C_r$ est la condition connue utilisée dans le premier récipient de référence, $\Delta P$ est la différence de pression entre les deux récipients et $P_r$ est la pression dans le premier récipient de référence.

Dans le cas où l'activité des microorganismes ne varie pas de façon linéaire avec la condition de culture inconnue à déterminer, on ne pourra pas utiliser le procédé de l'invention pour déterminer quantitativement cette condition de culture, mais on pourra néanmoins utiliser le procédé de l'invention pour détecter des variations de cette condition de culture, ce qui peut être également très intéressant pour effectuer des contrôles dans divers domaines de l'industrie.

L'invention a également pour objet un dispositif de mise en oeuvre du procédé décrit ci-dessus.

Ce dispositif comprend
- une enceinte thermostatée apte à recevoir un premier récipient et un second récipient ,
- des moyens pour supporter ce premier et ce second récipients dans l'enceinte,
- des moyens pour soumettre les deux récipients à une agitation dans les mêmes conditions,
- une première conduite reliant le premier récipient à des moyens de mesure de la différence de pression entre les deux récipients,
- une seconde conduite de volume identique à celui de la première conduite reliant le second récipient aux moyens de mesure de la différence de pression entre les deux récipients,
- des moyens pour relier respectivement de façon étanche la première conduite au premier récipient et la seconde conduite au second récipient, et
- des conduites munies de vannes pour relier chacun des récipients à l'atmosphère ambiante.

Le procédé et le dispositif de l'invention peuvent trouver de nombreuses applications dans divers domaines.

Ainsi, lorsque la condition différente de culture porte sur la présence de produits toxiques dans le milieu de culture, on peut utiliser l'invention pour détecter la présence de produit(s) toxique(s) dans des effluents aqueux, tels que des eaux de distribution, des effluents et autres déchets industriels, agricoles ou urbains, par exemple pour contrôler leur pollution en vue de rejets ou de traitements ultérieurs par voie biologiques.

Lorsque la condition de culture à déterminer porte sur le nombre de microorganismes, on peut utiliser l'invention pour contrôler des processus de fermentation dans diverses industries, par exemples contrôler la concentration en cellules d'une culture de levain dans l'industrie agroalimentaire.

Lorsque la condition de culture à déterminer est la concentration en substrat du milieu de culture, on peut utiliser l'invention dans la détermination de la concentration d'un substrat spécifique fermenté par la levure.

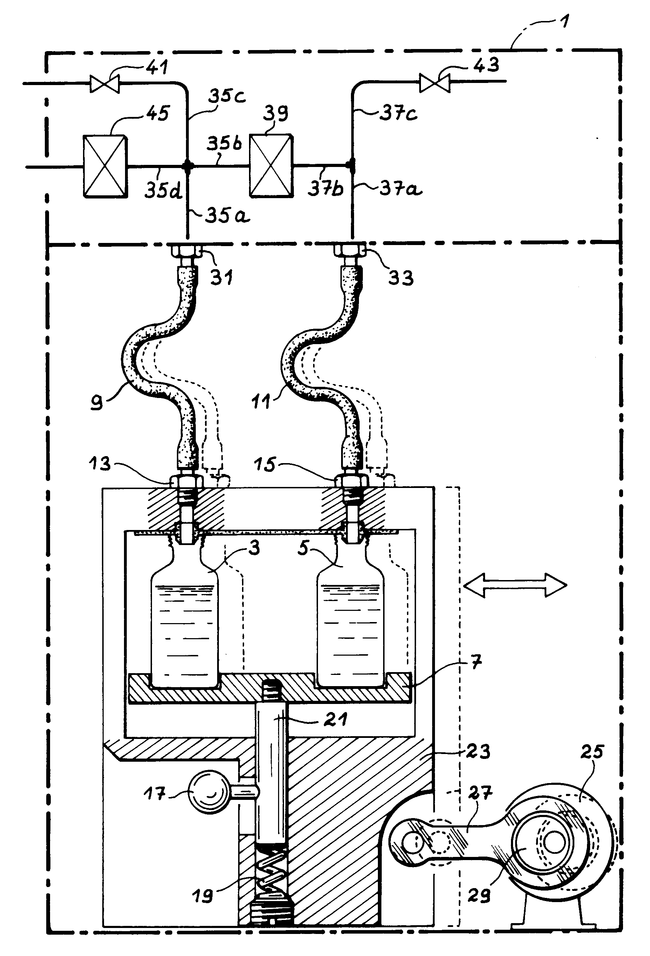

D'autres caractéristiques et avantages de l'invention apparaîtront mieux à la lecture de la description qui suit, donnée bien entendu à titre illustratif et non limitatif, en référence au dessin annexé.

La figure unique annexée est une représentation schématique en coupe verticale d'un dispositif conforme à l'invention.

Sur cette figure, on voit que le dispositif de l'invention comprend une enceinte thermostatée (1) à l'intérieur de laquelle on peut disposer le premier récipient de référence (3) et le second récipient (5) sur un plateau-support (7). Le plateau-support (7) peut prendre deux positions différentes dans le sens vertical dont une première position haute représentée sur le dessin. Dans cette position haute, le premier récipient (3) et le second récipient (5) sont reliés de façon étanche respectivement avec une première conduite (9) et une seconde conduite (11) flexibles par l'intermédiaire des raccords (13 et 15). La position basse du plateau-support (7) est obtenue en actionnant la poignée (17) vers le bas et en la déplaçant dans une rainure appropriée pour comprimer le ressort (19). Ce ressort (19) assure le maintien du plateau-support (7) en position haute par l'intermédiaire de la tige (21) qui coulisse dans un bâti (23) supportant également les raccords (13) et (15).

Dans cette position basse, le premier récipient (3) et le second récipient (5) sont désolidarisés des conduites flexibles (9) et (11), pour permettre leur remplissage et leur manutention.

Pour pouvoir soumettre à une agitation les cultures contenues dans les récipients (3) et (5), le plateau-support (7) de ces récipients qui est solidaire du bâti (23), peut se déplacer horizontalement avec le bâti (23), entre deux positions extrêmes (représentées en traits pleins et en tirets sur le dessin), sous l'action du moteur (25) par l'intermédiaire du système constitué par la bielle (27) et la came (29).

A leur partie supérieure le premier conduit flexible (9) et le second conduit flexible (11) sont reliés respectivement par les raccords (31) et (33) aux conduites (35a et 35b) et (37a et 37b) reliées à un capteur différentiel de pression (39). Les conduites (35a) et (37a) peuvent être également reliées à l'atmosphère ambiante, respectivement par l'intermédiaire des conduites (35c) et (37c) et des vannes (41) et (43).

Comme représenté sur le dessin, le dispositif comprend de plus un capteur différentiel de pression (45) pour mesurer la différence de pression entre le récipient (3) par l'intermédiaire des conduites (35d et 35a), et l'atmosphère ambiante.

Dans ce dispositif, le volume total de l'ensemble constitué par le récipient (3) et les conduites (9, 35a, 35b, 35c et 35d) est égal au volume total de l'ensemble constitué par le récipient (5) et les conduites (11, 37a, 37b et 37c).

Pour mettre en oeuvre le procédé de l'invention dans ce dispositif, on amène tout d'abord le plateau-support (7) en position basse en actionnant la poignée (17) afin de libérer le premier récipient (3) et le second récipient (5) des conduites (9) et (11), puis on introduit dans le premier récipient une culture de microorganismes servant de référence et dans le second récipient (5) une culture des mêmes microorganismes qui se différencie de la culture de référence du récipient (3) par une condition de culture telle que le nombre de microorganismes, la présence de produit(s) toxique(s) dans le milieu de culture, ou la concentration en substrat du milieu de culture. On actionne ensuite la poignée (17) pour faire revenir le plateau-support (7) en position haute et solidariser les récipients (3) et (5) respectivement avec les conduites (9) et (11) qui sont reliées à l'atmosphère ambiante, les vannes (41) et (43) étant ouvertes.

On ferme alors les vannes (41) et (43), puis on règle l'enceinte thermostatée (1) à la température voulue et on actionne le moteur (25) pour soumettre les deux récipients à une agitation et effectuer la culture dans les mêmes conditions.

Lorsque le capteur différentiel de pression (45) enregistre une augmentation de pression correspondant au seuil que l'on s'est fixé, on mesure la différence de pression enregistrée par le capteur (39) et on en déduit la condition de culture inconnue du récipient (5).

Avec le dispositif de l'invention, on peut ainsi garantir que les deux cultures sont effectuées dans les mêmes conditions (agitation, température), ce qui n'est pas toujours le cas lorsque l'on réalise successivement deux cultures de microorganismes.

Les exemples suivants illustrent l'utilisation du procédé de l'invention pour détecter la présence d'un produit toxique dans un effluent et pour déterminer la concentration en cellules d'une culture de levain.

**Exemple 1** : Détection de la présence d'un produit toxique dans un échantillon aqueux.

Dans cet exemple, on utilise la levure Saccharomyces $LyB_1$ déposée sous le n° I-965 à la Collection Nationale de Cultures de Microorganismes de l'Institut Pasteur pour détecter la présence d'un produit toxique dans un échantillon aqueux.

Dans ce but, on introduit dans le premier récipient environ $5.10^8$ cellules de levure dans 5ml d'un milieu de culture MFL constitué par de l'eau distillée contenant 20g/l de sucre fermentescible et 5g/l d'extrait de levure.

Dans le second récipient, on introduit également environ $5.10^8$ cellules de levure dans 5ml de milieu de culture MFL contenant les mêmes quantités de sucre fermentescible et d'extrait de levure que celui du premier récipient, mais dans lequel l'eau distillée a été remplacée par l'échantillon aqueux à analyser.

On réalise ensuite la fermentation simultanément dans les deux récipients en opérant à une température de 28°C, un pH de 4,4 et en soumettant les deux récipients à une agitation à une vitesse de 800 oscillations par minute. Dans ces conditions de fermentation optimale, la quantité d'anhydride carbonique produite dans le flacon de référence dépend de la concentration en levure et en substrat, de la température et de l'efficacité de l'agitation pour que le $CO_2$ formé soit aussitôt désorbé de la solution.

Comme le volume gazeux au-dessus de la solution dans le premier récipient est constant (10ml), cette production de $CO_2$ se traduit par un accroissement de pression $\Delta P_R$ que l'on peut détecter au moyen du capteur de pression (45).

Dans le second récipient, les paramètres de culture sont rigoureusement identiques à ceux du premier récipient (nombre de cellules de levure, quantité de substrat fermentable, température, agitation, volume de la solution) sauf en ce qui concerne la nature du solvant (eau) du milieu de culture, puisque celui-ci peut contenir des substances telles que des métaux lourds, des pesticides ou d'autres produits toxiques qui auront pour effet d'altérer ou d'inhiber le processus de fermentation.

Dans ces conditions, au bout du même temps, l'accroissement de pression $\Delta P_E$ au-dessus de la solution dans le second récipient où le volume gazeux est également de 10ml, sera égal ou inférieur à $\Delta P_R$ qui représente l'accroissement de pression dans le milieu de référence.

On peut définir ainsi un pourcentage de toxicité

$$T = \left(1 - \frac{\Delta P_R - \Delta P_E}{\Delta P_R} x\right) 100$$

Pour obtenir cette valeur de la toxicité, on mesure la différence de pression $(\Delta P_R - \Delta P_E)$ entre le premier et le second récipients lorsque $\Delta P_R$ atteint le seuil de $25.10^2 Pa$(25mbars), c'est-à-dire au bout de 5min environ.

**Exemple 2** : Mesure de la concentration en microorganismes d'un levain.

Pour effectuer cette mesure, on introduit dans le premier récipient (3) 5ml d'une solution contenant $5.10^5$ cellules de levure, ayant un pH de 4,5 et contenant du glucose et des extraits de levure.

Dans le second récipient (5), on introduit 4,5ml d'une solution à pH 4,4 contenant des quantités de glucose et d'extrait de levure identiques à celles de la solution introduite dans le premier récipient, puis on ajoute 0,5ml de l'échantillon de culture de levain.

On réalise ensuite la fermentation dans les deux récipients dans les mêmes conditions, à une température de 28°C, avec une agitation correspondant à 800 oscillations par minute.

Lorsque la différence de pression $\Delta P_R$ du capteur de pression (45) atteint la valeur de $25.10^2Pa$ (25mbars), on détermine la différence de pression ($\Delta P_R - \Delta P_E$) au moyen du capteur de pression (39).

A partir de cette valeur, on peut déterminer le nombre de cellules de la culture du second récipient à partir de la formule :

$$N_X = N_1 \left( 1 - \frac{\Delta P_R - \Delta P_E}{\Delta P_R} \right)$$

dans laquelle $N_1$ représente le nombre de cellules dans le premier récipient ($5.10^5$). A partir de $N_x$ on peut déterminer la concentration en cellules du levain qui est égale à $N_x/0,5 \times 10^{-3}$.

## Revendications

1. Procédé pour déterminer une condition de culture inconnue d'une culture de microorganismes donnant un dégagement de gaz, caractérisé en ce qu'il comprend les étapes suivantes :
   a) cultiver dans un premier récipient de référence fermé de façon étanche, une population desdits microorganismes dans des conditions de culture connues,
   b) cultiver dans un second récipient fermé de façon étanche, ayant le même volume que le premier récipient, une population des mêmes microorganismes dans des conditions de culture identiques, sauf pour la condition de culture inconnue, différente,
   c) mesurer la différence de pression gazeuse entre le premier et le second récipients, et
   d) déterminer à partir de cette différence de pression, la condition de culture inconnue, différente, utilisée dans le second récipient.

2. Procédé selon la revendication 1, caractérisé en ce que l'on détermine la différence de pression entre les deux récipients lorsque la pression dans le premier récipient atteint un seuil prédéterminé.

3. Procédé selon la revendication 2, caractérisé en ce que ce seuil correspond à une augmentation de pression dans le premier récipient de $25.10^2Pa$.

4. Procédé selon l'une quelconque des revendications 1 à 3, caractérisé en ce que les microorganismes sont des levures capables de réaliser une fermentation éthanolique.

5. Procédé selon l'une quelconque des revendications 1 à 4, caractérisé en ce que la condition de culture inconnue, différente est la concentration en produit toxique du milieu de culture.

6. Procédé selon l'une quelconque des revendications 1 à 4, caractérisé en ce que la condition de culture inconnue, différente est le nombre de microorganismes de la culture.

7. Procédé selon l'une quelconque des revendications 1 à 4, caractérisé en ce que la condition de culture inconnue, différente est la concentration en substrat du milieu de culture.

8. Utilisation du procédé selon la revendication 5 pour détecter la présence de produit toxique dans un effluent aqueux.

9. Utilisation du procédé selon la revendication 6, pour mesurer la concentration en cellules d'une culture de levain.

10. Dispositif pour la mise en oeuvre du procédé selon l'une quelconque des revendications 1 à 7, caractérisé en ce qu'il comprend :
    - une enceinte thermostatée (1) apte à recevoir un premier récipient (3) et un second récipient (5),
    - des moyens (7) pour supporter ce premier et ce second récipients dans l'enceinte,
    - des moyens (23, 25, 27, 29) pour soumettre les deux récipients à une agitation dans les mêmes conditions,
    - une première conduite (9) reliant le premier récipient (3) à des moyens de mesure (39) de la différence de pression entre les deux récipients,
    - une seconde conduite (11) de volume identique à celui de la première conduite, reliant le second récipient (5) aux moyens de mesure (39) de la différence de pression entre les deux récipients,
    - des moyens pour relier respectivement de façon étanche la première conduite au premier récipient et la seconde conduite au second récipient, et
    - des conduites munies de vannes (41, 43) pour relier chacun des récipients à l'atmosphère ambiante.

11. Dispositif selon la revendication 10, caractérisé en ce qu'il comprend de plus des moyens (45) pour mesurer la différence de pression entre le premier récipient et l'atmosphère ambiante.

Office européen
des brevets

## RAPPORT DE RECHERCHE EUROPEENNE

Numero de la demande

EP 94 40 0302

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| Y | EP-A-0 063 744 (KERNFORSCHUNGSANLAGE JÜLICH GESELLSCHAFT MIT BESCHRÄNKTER HAFTUNG)<br>* abrégé *<br>--- | 1,4,5,10 | C12M1/34<br>C12M1/02 |
| D,Y | WO-A-92 01064 (BREVATOME)<br>* le document en entier *<br>--- | 1,4,5,10 | |
| Y | US-A-4 311 794 (J.L. MELNICK & C. WALLIS)<br><br>* abrégé *<br>* colonne 7, ligne 20 - colonne 8, ligne 12 *<br>--- | 1-3,6,<br>10,11 | |
| Y | EP-A-0 184 260 (UNILEVER NV)<br><br>* le document en entier *<br>--- | 1-3,6,<br>10,11 | |
| A | DE-C-40 17 754 (P. OEHLERT)<br>--- | | |
| A | Dynatech Laboratories Ltd. Product Informa "Shaker Incubator", 28.7.83, Dynatech Labo es Ltd, Sussex, England.<br>----- | | **DOMAINES TECHNIQUES RECHERCHES (Int.Cl.5)**<br><br>C12M |

Le présent rapport a été établi pour toutes les revendications

| Lieu de la recherche | Date d'achèvement de la recherche | Examinateur |
|---|---|---|
| LA HAYE | 20 Mai 1994 | Bevan, S |